Europäisches Patentamt

European Patent Office

Office européen des brevets

(19)

(11) Numéro de publication : **0 001 945**
**B1**

(12) ## FASCICULE DE BREVET EUROPÉEN

(45) Date de publication du fascicule du brevet :
25.03.81

(21) Numéro de dépôt : **78400143.0**

(22) Date de dépôt : **23.10.78**

(51) Int. Cl.³ : **C 12 P 19/04, A 61 K 31/715**

(54) Nouveaux polysaccharides extraits de corps microbiens d'Haemophilus Influenzae, leur procédé de préparation et les compositions pharmaceutiques les renfermant.

(30) Priorité : **27.10.77 FR 7732416**

(43) Date de publication de la demande :
**16.05.79 (Bulletin 79/10)**

(45) Mention de la délivrance du brevet :
**25.03.81 Bulletin 81/12**

(84) Etats contractants désignés :
**BE CH DE GB NL SE**

(56) Documents cités :
**FR - A - 2 043 475**
**GB - A - 725 938**

(73) Titulaire : **ROUSSEL-UCLAF**
**102, route de Noisy Boîte postale no.9**
**F-93230-Romainville (FR)**

(72) Inventeur : **Brossard Claudine**
**5 rue Paul Doumer**
**F-95520-Osny (FR)**
Inventeur : **Henry Martine**
**6 rue Jean Jaurès**
**F-78330-Fontenay le Fleury (FR)**
Inventeur : **Raichvarg Denis**
**11 chemin des Grives**
**F-93800-Epinay sur Seine (FR)**

(74) Mandataire : **Vieillefosse, Jean-Claude et al**
**boîte postale no 9 102, route de Noisy**
**F-93230 Romainville (FR)**

Imprimerie Jouve, 17, rue du Louvre, 75001 Paris, France

Nouveaux polysaccharides extraits de corps microbiens d'Haemophilus Influenzae,
leur procédé de préparation et les compositions pharmaceutiques les renfermant.

La présente invention concerne de nouveaux polysaccharides extraits de corps microbiens d'Haemophilus Influenzae, leur procédé de préparation et les compositions pharmaceutiques les renfermant.

Le brevet français n° 2.043.475 a déjà décrit des α et β-glycoprotéines d'origine microbienne, ces glycoprotéines ont été obtenues par lyse des cultures microbiennes, délipidation du lysat, déprotidation subséquente, purification sélective d'une fraction glycoprotéique puis purification.

Le brevet anglais 725.938 a décrit un procédé de préparation de polysaccharides pyrogènes à partir de microorganismes, par extraction des micro-organismes par la méthode phénol-eau, refroidissement, et isolation des polysaccharides à partir de la phase aqueuse, lesdits polysaccharides étant capables d'acquérir des propriétés antigéniques par combinaison à une protéine bactérienne.

Des polysaccharides extraits de certaines souches d'Haemophilus Influenzae sont déjà décrits dans la littérature.

C'est ainsi qu'un article de 1971 publié dans le « Journal of Immunology » (Vol. 107, n° 4, Octobre 1971, pages 1071-1080) décrit la préparation de tels polysaccharides à partir de capsules bactériennes d'une souche d'Haemophilus Influenzae de sérotype b. Selon cette publication, la majorité des effets causés par Haemophilus Influenzae, tant chez l'homme que chez l'enfant, et notamment dans le cas des méningites, serait due à la présence d'une toxine de nature polysaccharidique présente dans les capsules d'Haemophilus Influenzae de sérotype b.

L'objet de cette publication est ainsi de préparer un extrait capsulaire puis d'éliminer de cet extrait l'endotoxine et les substances pyrogéniques par déprotéinisation, afin de trouver un remède aux réactions précitées.

Pour effectuer une telle préparation, les auteurs cherchent à préparer, de manière prédominante, la forme capsulaire d'Haemophilus Influenzae pour en isoler le produit recherché. Schématiquement ils procèdent par traitement au formaldéhyde suivi d'une précipitation à l'éthanol puis d'une élution sur colonne.

Selon les indications figurant dans l'article précité, le polysaccharide obtenu est constitué par un polyribose phosphate possédant des liaisons glycosidiques entre les atomes $C_1$ et $C_4$ de la forme furanoside du D-ribose.

Selon cet article, le polysaccharide obtenu a perdu ses propriétés toxiques, tout en conservant ses propriétés antigéniques et immunisantes spécifiques vis-à-vis d'Haemophilus Influenzae.

La demanderesse a cherché de son côté à isoler, non plus à partir des capsules d'Haemophilus Influenzae, mais à partir des germes eux-mêmes, une toxine détoxifiée de nature polysaccharidique pouvant présenter des propriétés antigéniques et immunostimulantes ainsi qu'une bonne tolérance qui puissent être utilisées dans le traitement ou la prévention d'affections à Haemophilus Influenzae, ainsi que dans le traitement de diverses affections respiratoires ou de bronchites ; elle a ainsi trouvé de nouveaux polysaccharides isolés de corps microbiens d'Haemophilus Influenzae qui présentent les critères recherchés.

La présente demande a ainsi pour objet de nouveaux polysaccharides caractérisés en ce qu'ils sont extraits de corps microbiens d'Haemophilus Influenzae, en ce qu'ils possédent un poids moléculaire apparent supérieur à 200 000 et en ce qu'ils sont constitués principalement de galactose, de glucose et de mannose.

Le poids moléculaire apparent est le poids moléculaire déterminé au moyen d'un gel poreux étalonné à l'aide de solutions macromoléculaires connues. Parmi les gels poreux étalonnés permettant le tamisage moléculaire, on peut retenir les gels commercialisés sous la désignation Sephadex (gels Sephadex G 200) et les gels d'agarose (Sepharose 4B).

L'expression polysaccharides constitués principalement de galactose, de glucose et de mannose signifie que ces polysaccharides renferment au moins 40 % à 50 % de ces oses.

Parmi les polysaccharides de la présente invention, on retient notamment les polysaccharides caractérisés en ce qu'ils renferment en outre une faible proportion d'osamines.

Selon l'invention, la faible proportion d'osamines entrant dans la composition des polysaccharides est constituée par de la glucosamine.

La teneur en osamines déterminée (selon la méthode d'Elson Morgan) est pour les polysaccharides de la présente invention voisine de 2 %.

Les polysaccharides de la présente demande possédent une teneur en oses neutres, c'est-à-dire, en galactose, glucose et mannose, voisine de 44,5 % (dosage effectué par la méthode à l'Orcinol corrigé).

Ces produits renferment des traces de sucres migrant par chromatographie au niveau des pentoses mais ne renferment pas d'Heptoses, ni d'acides uroniques.

Ces produits ne renferment pas non plus d'acide diaminopimélique. L'absence d'acide diaminopimélique dans les polysaccharides tels que définis ci-dessus indique qu'il n'existe pas de contamination par les peptido-glycanes de la paroi membranaire.

Parmi les polysaccharides tels que définis ci-dessus, on retient plus particulièrement ceux obtenus à partir des souches d'Haemophilus Influenzae déposées à l'Institut Pasteur à Paris, sous les n° 52 151 et 5 481.

Parmi ces dernières souches, on retient plus particulièrement la souche déposée à l'Institut Pasteur à Paris sous le n° 52 151.

Ces souches appartiennent au sérotype a.

L'invention a également pour objet un procédé de préparation des polysaccharides tels que définis ci-dessus, caractérisé en ce que l'on cultive, sur milieu solide ou liquide, une souche microbienne d'Haemophilus Influenzae, récolte après complet développement les corps microbiens, procède à une extraction phénolique des corps microbiens lavés, recueille la phase phénolique, effectue un retour en phase aqueuse pour obtenir un produit brut impur que l'on purifie, puis effectue une hydrolyse ménagée du produit purifié ainsi obtenu, pour en éliminer les acides gras combinés et isole les polysaccharides recherchés.

Les souches d'Haemophilus Influenzae peuvent notamment être cultivées dans les milieux liquides agités, dans des conditions aérobies. Les milieux de culture utilisés sont usuels pour de telles souches. Ces milieux peuvent par exemple comprendre des extraits de viande, de la peptone de caséine, des autolysats de levure, de la peptone papaïnique de soja, des sucres, des éléments minéraux, de l'hémine, du coenzyme I et de l'eau distillée.

Selon l'invention et afin d'obtenir un taux optimal en polysaccharides, il y a lieu de récolter les germes après complet développement des corps microbiens soit après environ 6 heures, à une température de 37 °C.

Les corps microbiens peuvent alors être lavés.

Selon le procédé, objet de l'invention, l'extraction phénolique des corps microbiens lavés est effectuée au moyen d'une solution aqueuse phénolique en opérant à une température voisine de 65 °C.

La purification du produit brut impur consiste à précipiter les acides nucléiques par du sulfate de Streptomycine.

Le sulfate de streptomycine présent dans le surnageant peut avantageusement être éliminé par dialyse sur une membrane poreuse. La membrane poreuse peut se présenter sous la forme d'une cartouche de fibres creuses telles que les fibres « Hollow fiber » $H_1DP_{10}$ possédant un seuil de rétention de substances dont le poids moléculaire est supérieur à 10 000.

Le produit ainsi purifié peut alors avantageusement être lyophilisé.

Le procédé, objet de la présente invention, est enfin caractérisé en ce que l'hydrolyse ménagée du produit purifié est effectué à chaud au sein d'un solvant hydro-organique, en présence d'une résine échangeuse d'ions.

L'hydrolyse ménagée du produit purifié est contrôlée par la réaction de Schwartzman (mise en évidence chez le lapin d'une hypersensibilité locale non spécifique se traduisant par une altération de l'endothélium des vaisseaux cutanés et une infiltration leucocytaire de la paroi des petits vaisseaux).

L'hydrolyse ménagée du produit purifié est avantageusement effectuée à la température de reflux, au sein d'un mélange eau-chloroforme, en présence d'une résine échangeuse d'ions telle que la résine commercialisée sous la marque « Dowex 50W X 8, 200-400 mesh $H^+$ n° 41 631 ».

L'hydrolyse ménagée est arrêtée lorsque la réaction de Schwartzman devient négative. Dans les conditions de l'essai, cette réaction devient négative au bout de 15 heures environ. Le produit obtenu peut alors avantageusement être lyophilisé.

La présente demande a encore pour objet les polysaccharides extraits de corps microbiens d'Haemophilus Influenzae, obtenus par le procédé décrit ci-dessus.

Les produits, objet de la présente invention, possèdent de très intéressantes propriétés pharmacologiques, ils sont doués notamment de remarquables propriétés antigéniques et immunostimulantes. Ils ont de plus une très bonne tolérance.

Ces propriétés sont illustrées plus loin dans la partie expérimentale.

Ces propriétés justifient l'utilisation des polysaccharides objet de la présente demande, à titre de médicaments.

Parmi les médicaments de l'invention, on peut citer, en particulier, ceux caractérisés en ce qu'ils sont constitués par les nouveaux polysaccharides tels qu'obtenus par le procédé de l'invention.

Les médicaments selon l'invention trouvent, par exemple, leur emploi dans le traitement ou la prévention des maladies à Haemophilus Influenzae, dans le traitement ou la prévention des affections respiratoires, des bronchites, des bronchites chroniques.

La dose usuelle, variable selon le produit utilisé, le sujet traité et l'affection en cause peut être, par exemple, de 1 à 100 microgrammes par jour, par voie perlinguale chez l'homme.

A titre de médicaments, les polysaccharides, objet de la présente demande, peuvent être incorporés dans des compositions pharmaceutiques destinées à la voie digestive, parentérale ou locale et l'invention a également pour objet lesdites compositions pharmaceutiques.

Ces compositions pharmaceutiques sont préparées selon les méthodes usuelles et peuvent être, par exemple, solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou

3

végétale, les dérivés paraffiniques, les glycols, les divers agents mouillants, dispersants ou émulsifiants, les conservateurs.

Il va être donné maintenant, à titre non limitatif, des exemples de mise en œuvre de l'invention.

Exemple 1

Stade A : Culture

Par simple mélange des différents constituants, on prépare un milieu nutritif répondant à la formule :

— extrait de viande ............................................................................................................... 5 g
— chlorure de sodium ........................................................................................................... 5 g
— peptone de caséine ........................................................................................................... 5 g
— autolysat de levure ........................................................................................................... 5 g
— phosphate bipotassique .................................................................................................... 3,5 g
— phosphate monopotassique .............................................................................................. 1,5 g
— peptone papaïnique de soja .............................................................................................. 20 g
— eau distillée ............................................... q.s.p. ......................................................... 1.000 ml

On ajuste le pH à 7,4-7,6 et stérilise le milieu. On ensemence le milieu à l'aide d'une souche d'Haemophilus Influenzae (souche Institut Pasteur sérotype a n° 52 151) et ajoute 10 g de glucose, 1 mg d'hémine et 1,5 mg de coenzyme I. On maintient 24 heures à l'étuve à 37 °C.

On introduit l'inoculum obtenu ci-dessus dans 10 litres de milieu nutritif possédant la même composition que celle indiquée ci-dessus et renfermant les mêmes additifs.

Après 16 heures à 37 °C, on obtient une préculture que l'on utilise pour ensemencer 80 litres d'un milieu nutritif possédant la même composition que celle indiquée ci-dessus, puis ajoute 800 g de glucose, 120 mg d'hémine et 160 mg de coenzyme I ainsi que 2,8 litres de solution mère de composition suivante pour 10 litres : (1 litre d'extrait de levure, 400 g de glucose dans 1 litre d'eau, eau q.s. pour 10 litres, pH ajusté à 8).

On laisse fermenter pendant 6 heures puis centrifuge en continu à 30 000 tours/minute. On recueille 130 g de germes humides.

Stade B : Extraction des germes

A 100 g de germes humides obtenus ci-dessus, on ajoute 870 ml d'eau permutée chauffée à 68 °C et 870 ml de phénol à 90 % également chauffé à 68 °C. On agite à 2 000 tours/minute et laisse le mélange à 68 °C pendant 30 minutes.

On garde le mélange 12 heures à + 4 °C, sépare les deux phases phénol-eau par centrifugation en continu (sur centrifugeuse Westfalia), puis clarifie la phase aqueuse.

On effectue une seconde extraction de la phase phénolique en ajoutant les boues obtenues lors de la centrifugation précédente et de l'eau permutée afin d'obtenir le même volume que lors de la première extraction. On agite comme précédemment 30 minutes à 68 °C, laisse 12 heures à 4 °C et centrifuge.

On recueille les deux phases aqueuses provenant des extractions et les dialyse pendant 4 jours pour éliminer le phénol. Après disparition du phénol, on réunit les phases aqueuses, les concentre sur membrane Amicon (membrane $H_1DP_{10}$ possédant un seuil de rétention de substances dont le poids moléculaire est supérieur à 10 000), en réduisant le volume à 3,5 litres.

On centrifuge à + 4 °C à 12 000 tours/minute pendant 20 minutes, recueille la phase aqueuse et lyophilise.

On obtient 6 g d'un produit brut se présentant sous la forme d'une poudre blanche floconneuse.

Stade C : Purification du produit brut

A 1 200 ml d'une solution renfermant 5 mg/ml de produit brut obtenu ci-dessus, on ajoute lentement, en 45 minutes environ et sous vive agitation, 240 ml d'une solution de sulfate de streptomycine à 2,5 %. On précipite ainsi les acides nucléiques.

On laisse reposer 15 minutes et centrifuge à 12 000 tours/minute pendant 20 minutes à 4 °C. On recueille la phase aqueuse, élimine le sulfate de streptomycine du surnageant par dialyse sur membrane poreuse (Hollow Fiber cartouche $H_1DP_{10}$), possédant un seuil de rétention de substances dont le poids moléculaire est supérieur à 10 000.

On lyophilise alors le produit purifié ainsi obtenu et recueille 1,3 g de produit purifié.

Analyse : C % 40,26  H % 6,84  N % 6,53

Teneurs en :

| | | |
|---|---|---|
| — Protéines | (méthode de Lowry) | 9,5 % |
| — Oses neutres | (méthode à l'orcinol) | 41 % |
| — Oses neutres | (méthode à l'orcinol corrigé) | 35 % |
| — Acides uroniques | (carbazol corrigé) | 6,8 % |
| — Osamines | (méthode Elson Morgan) | 5,6 % |
| — Pentoses | (méthode Bial) | 1 % |

Stade D : Hydrolyse

On dissout dans 650 ml d'eau les 1,3 g de produit purifié obtenu ci-dessus, agite à + 4 °C pendant 12 heures, puis centrifuge pendant 20 minutes.

On recueille le surnageant, ajoute 650 ml de chloroforme et 310 g de résine Dowex (50 W X 8-200-400 mesh H$^+$) essorée sur buchner.

On porte le mélange au reflux pendant 15 heures (temps nécessaire pour obtenir que la réaction de Schwartzman soit négative) élimine le chloroforme par concentration sous vide à 40 °C, puis lyophilise.

On recueille 0,65 g de polysaccharides extraits de corps microbiens d'Haemophilus Influenzae.

Analyse : C % 40,02  H % 6,53  N % 0,90

Teneurs en :

| | | |
|---|---|---|
| — Protéines | (méthode de Lowry) | 1 % |
| — Oses neutres | (méthode à l'orcinol corrigé) | 44,5 % |
| — Acides uroniques | (carbazol corrigé) | 0 % |
| — Osamines | (méthode Elson Morgan) | 2 % |
| — Pentoses | (méthode Bial) | 0,8 % |
| — Heptoses | (méthode de Dische) | 0 % |

— Recherche de l'acide diaminopimélique : négative.
— Identification des oses neutres (par chromatographie sur acétate de cellulose, gel de silice, papier), présence de galactose, glucose, mannose.
— Identification de l'osamine par électrophorèse : présence de glucosamine.

Exemple 2 : Compositions pharmaceutiques

On a préparé des comprimés sublinguaux répondant à la formule :

| | |
|---|---|
| — polysaccharides obtenus à l'exemple 1 ................................................ | 25 µg |
| — excipient q.s. pour un comprimé terminé à ................................................ | 100 mg |

(Détail de l'excipient : gomme, gomme adragante, lactose, talc, amidon, stéarate de magnésium).

Exemple 3 : Compositions pharmaceutiques

On a préparé un soluté injectable répondant à la formule :

| | |
|---|---|
| — polysaccharides obtenus à l'exemple 1 ................................................ | 25 µg |
| — soluté injectable aqueux isotonique ................................................ | 2 cm$^3$ |

Etude pharmacologique

I. Détermination de l'activité antigénique

L'activité antigénique du produit obtenu à l'exemple 1 est mise en évidence vis-à-vis d'un sérum anti-Haemophilus Influenzae type a.

Cette mise en évidence est effectuée sur des tests classiques utilisés en immunologie, à savoir sur le « ring test », par hémagglutination passive, par agglutination des germes, par la technique d'Ouchterlony et en établissant une courbe de précipitation.

Dans ces différents tests, le produit obtenu à l'exemple 1 a donné des titres d'hémagglutination passive allant jusqu'au 1/128 et 1/256ème.

II. Mise en évidence du pouvoir immunogène

Des lapins Fauve de Bourgogne sont répartis en 6 lots. Chaque lot reçoit par voie intercostale et intraveineuse une dose de produit de l'exemple 1 égale à 1, 10, 100 µg/kg. Une série de trois lapins reçoit le produit de l'exemple 1 sans adjuvant ; l'autre série est immunisée par le produit de l'exemple 1 plus l'adjuvant complet de Freund. Une injection de produit est faite tous les 30 jours. Les prélèvements de sang sont effectués à :

$t_0 - t_{+38j} - t_{+47j} - t_{+74j} - t_{+76j} - t_{+101j} - t_{+105j} - t_{+130j}$.

La présence d'anticorps spécifiques est détectée à partir du 38ème jour et augmente après les rappels, les titres d'hémagglutination trouvés après cette immunisation étant de 1/128 et 1/256. Les sérums ont été étudiés par les techniques du « Ring-test », de l'agglutination et de la précipitation quantitative.

On ne note aucune différence entre les animaux vaccinés par le produit de l'exemple 1 seul et ceux vaccinés par le produit de l'exemple 1 + adjuvant de Freund.


III. Opsonines

Cette technique a pour objet de mettre en évidence par méthode colorimétrique l'aptitude des leucocytes à phagocyter les bactéries.

On admet lors de cette détermination que 0 % d'opsonisation est égal à une densité optique de 0,050 et que 100 % d'opsonisation est égal à une densité optique maximale de 0,250.

Après avoir effectué le calcul, on a trouvé que le pourcentage d'opsonisation chez les animaux immunisés par le produit de l'exemple 1 varie entre 50 et 70 % (chez les animaux témoins, le pourcentage d'opsonisation est de 0 %).


IV. Mise en évidence du pouvoir bactéricide du sérum

Du sérum de lapins immunisés par le produit de l'exemple 1 est mis en contact avec une culture d'Haemophilus Influenzae à différentes dilutions. Après ensemencement sur un milieu gélosé et culture, on compte le nombre de colonies vivantes avec, pour témoin :
— lapin avant l'immunisation t = 0
(témoin positif = 0 % de mortalité des bactéries)
— du sérum de lapin vacciné avec les germes Haemophilus Influenzae
(témoin négatif = 100 % de mortalité).
On peut noter une augmentation très régulière du pouvoir bactéricide en fonction du temps.

$t_0$ = 0 % de mortalité des bactéries
$t_{38\ jours}$ = 0 %
$t_{47\ jours}$ = 0 %
$t_{52\ jours}$ = 80 % de mortalité des bactéries
$t_{74\ jours}$ ⎫
⎬ compris entre 75 et 80 %
$t_{76\ jours}$ ⎭
$t_{101\ jours}$ = 83 %
$t_{130\ jours}$ = 100 %
témoin sérum anti-H.I. = 100 %

Ce test montre l'activité spécifique du produit objet de la présente invention.


V. Neutralisation de l'activité endotoxinique au niveau local

Le produit de l'exemple 1 administré chez le lapin antagonise l'activité locale d'Haemophilus Influenzae en neutralisant l'activité d'un produit déclenchant une réaction locale (réaction de Schwartzman).


VI. Etude de la toxicité aiguë

La toxicité aiguë a été déterminée sur un lot de 10 souris. Après administration par voie intrapéritonéale, la dose léthale 50(DL50) du produit de l'exemple 1 a été trouvée comprise entre 65 et 110 mg/kg.


**Revendications**

1. Polysaccharides caractérisés en ce qu'ils sont extraits de corps microbiens d'Haemophilus Influenzae, en ce qu'ils possèdent un poids moléculaire apparent supérieur à 200 000, en ce qu'ils sont constitués d'au moins 40 à 50 % de galactose, de glucose et de mannose.

2. Polysaccharides tels que définis à la revendication 1, caractérisés en ce qu'ils renferment, en outre, environ 2 % d'osamines.

3. Polysaccharides tels que définis à la revendication 2, caractérisés en ce que les osamines sont constituées par de la glucosamine.

4. Polysaccharides tels que définis à la revendication 1, 2 ou 3, caractérisés en ce que les corps microbiens d'Haemophilus Influenzae sont issus des souches déposées à l'Institut Pasteur à Paris sous les nos 52 151 et 5 481.

5. Polysaccharides tels que définis à la revendication 4, caractérisés en ce que les corps microbiens d'Haemophilus Influenzae sont issus de la souche déposée à l'Institut Pasteur à Paris sous le n° 52 151.

6. Procédé de préparation des polysaccharides tels que définis à la revendication 1, caractérisé en ce que l'on cultive, sur milieu solide ou liquide, une souche microbienne d'Haemophilus Influenzae, récolte après complet développement les corps microbiens, procède à une extraction phénolique des corps microbiens lavés, recueille la phase phénolique, effectue un retour en phase aqueuse pour obtenir un produit brut impur que l'on purifie, puis effectue une hydorlyse ménagée du produit purifié ainsi obtenu, pour en éliminer les acides gras combinés et isole les polysaccharides recherchés.

7. Procédé selon la revendication 6, caractérisé en ce que l'extraction phénolique des corps microbiens lavés est effectuée au moyen d'une solution aqueuse phénolique en opérant à une température voisine de 65 °C.

8. Procédé selon la revendication 6 ou 7, caractérisé en ce que la purification du produit brut impur consiste à précipiter les acides nucléiques par du sulfate de streptomycine.

9. Procédé selon l'une quelconque des revendications 6, 7 ou 8, caractérisé en ce que l'hydrolyse ménagée du produit purifié est effectuée à chaud au sein d'un solvant hydroorganique, en présence d'une résine échangeuse d'ions.

10. Compositions pharmaceutiques renfermant, à titre de principe actif, les polysaccharides tels que définis à la revendication 1.

11. Compositions pharmaceutiques renfermant, à titre de principe actif, les polysaccharides tels que définis à l'une quelconque des revendications 2, 3, 4 ou 5.


**Ansprüche**

1. Polysaccharide, dadurch gekennzeichnet, daß sie Mikrobenkörperextrakte von Haemophilus influenzae sind, daß sie ein scheinbares Molekulargewicht von größer als 200 000 besitzen und daß sie wenigstens zu 40 bis 50 % aus Galaktose, Glucose und Mannose bestehen.

2. Polysaccharide gemäß Anspruch 1, dadurch gekennzeichnet, daß sie außerdem etwa 2 % Osamine umfassen.

3. Polysaccharide gemäß Anspruch 2, dadurch gekennzeichnet, daß die Osamine aus Glucosamin bestehen.

4. Polysaccharide gemäß Anspruch 1, 2 oder 3, dadurch gekennzeichnet, daß die Mikrobenkörper von Haemophilus influenzae den in dem Pasteur-Institut in Paris unter den Nummern 52 151 und 5 481 hinterlegten Stämmen entstammen.

5. Polysaccharide gemäß Anspruch 4, dadurch gekennzeichnet, daß die Mikrobenkörper von Haemophilus influenzae den in dem Pasteur-Institut in Paris unter der Nr. 52 151 hinterlegten Stamm entstammen.

6. Verfahren zur Herstellung neuer Polysaccharide gemäß Anspruch 1, dadurch gekennzeichnet, daß man auf festem oder flüssigem Milieu einen Mikrobenstamm von Haemophilus influenzae kultiviert, nach der vollständigen Entwicklung die Mikrobenkörper gewinnt, eine Phenolextraktion der gewaschenen Mikrobenkörper vornimmt, die Phenolphase gewinnt, erneut in die wäßrige Phase überführt, um ein unreines Rohprodukt zu erhalten, das man reinigt, anschließend eine schonende Hydrolyse des so erhaltenen gereinigten Produkts vornimmt, un hieraus die kombinierten Fettsäuren zu entfernen, und die gewünschten Polysaccharide isoliert.

7. Verfahren gemäß Anspruch 6, dadurch gekennzeichnet, daß die Phenolextraktion der gewaschenen Mikrobenkörper mit Hilfe einer wäßrigen phenolischen Lösung durchgeführt wird, wobei man bei einer Temperatur von etwa 65 °C arbeitet.

8. Verfahren gemäß Anspruch 6 oder 7, dadurch gekennzeichnet, daß die Reinigung des unreinen Rohprodukts darin besteht, die Nucleinsäuren mit Streptomycinsulfat auszufällen.

9. Verfahren gemäß einem der Ansprüche 6, 7 oder 8, dadurch gekennzeichnet, daß die schonende Hydrolyse des gereinigten Produkts in der Wärme in einem wäßrig-organischen Lösungsmittelmedium in Gegenwart eines Ionenaustauscherharzes durchgeführt wird.

10. Pharmazeutische Zusammensetzungen, enthaltend als Wirkstoff die Polysaccharide gemäß Anspruch 1.

11. Pharmazeutische Zusammensetzungen, enthaltend als Wirkstoff die Polysaccharide gemäß einem der Ansprüche 2, 3, 4 oder 5.

**Claims**

1. Polysaccharides, characterised in that they are extracted from microbial bodies of Haemophilus Influenzae, in that they have an apparent molecular weight greater than 200 000 and in that they are constituted by at least 40 to 50 % of galactose, glucose and mannose.

2. Polysaccharides as defined in Claim 1, characterised in that they contain, in addition, about 2 % of osamines.

3. Polysaccharides as defined in Claim 2, characterised in that the osamines are constituted by glucosamine.

4. Polysaccharides as defined in Claim 1, 2 or 3, characterised in that the microbial bodies of Haemophilus Influenzae have come from strains deposited at the Institut Pasteur in Paris under the numbers 52 151 and 5 481.

5. Polysaccharides as defined in Claim 4, characterised in that the microbial bodies of Haemophilus Influenzae have come from the strain deposited at the Institut Pasteur in Paris under the number 52 151.

6. Process for preparing the new polysaccharides as defined in Claim 1, characterised in that a microbial strain of Haemophilus Influenzae is cultivated on solid or liquid medium, the microbial bodies are harvested after complete development, phenolic extraction of the washed microbial bodies is then carried out, the phenolic phase is recovered, a return to the aqueous phase is brought about to obtain an impure crude product which is purified, then careful hydrolysis of the purified product thus obtained is carried out to eliminate therefrom the combined fatty acids and the polysaccharides sought are isolated.

7. Process according to Claim 6, characterised in that the phenolic extraction of the washed microbial bodies is carried out by means of an aqueous phenolic solution, work being carried out at a temperature close to 65 °C.

8. Process according to Claim 6 or 7, characterised in that the purification of the impure crude product consists in precipitating the nucleic acids with streptomycin sulphate.

9. Process according to any one of Claims 6, 7 or 8, characterised in that the careful hydrolysis of the purified product is carried out with heating in an aqueous organic solvent, in the presence of an ion exchange resin.

10. Pharmaceutical compositions containing, as active principle, the polysaccharides as defined in Claim 1.

11. Pharmaceutical compositions containing, as active principle, the polysaccharides as defined in any one of Claims 2, 3, 4 or 5.